# EUROPÄISCHE PATENTSCHRIFT

(11) **EP 3 846 890 B1**
(45) Veröffentlichungstag und Bekanntmachung des Hinweises auf die Patenterteilung: **08.11.2023**
(21) Anmeldenummer: 18810959.9
(22) Anmeldetag: 22.11.2018
(51) Int. Cl.: A61M 16/08, A61M 16/10, A61M 39/10

(54) **BEHEIZBARER BEATMUNGSSCHLAUCH SOWIE VERFAHREN ZUR HERSTELLUNG DESSELBEN**
HEATABLE BREATHING TUBE AND METHOD FOR THE PRODUCTION THEREOF
TUBE RESPIRATOIRE CHAUFFABLE ET SON PROCÉDÉ DE PRODUCTION

(43) Veröffentlichungstag der Anmeldung: 14.07.2021
(73) Patentinhaber: WILAmed GmbH, 91126 Kammerstein (DE)
(72) Erfinder: KLINGER, Miriam, 91126 Schwabach (DE)
(74) Vertreter: Stippl Patentanwälte
(86) Internationale Anmeldenummer: PCT/EP2018/082224
(87) Internationale Veröffentlichungsnummer: WO 2020/104030

(56) Entgegenhaltungen:
- US-A- 4 354 051
- US-A1- 2003 183 294
- US-A1- 2014 202 462
- US-B1- 6 367 510

## Beschreibung

Die vorliegende Erfindung betrifft einen beheizbaren als Verbundschlauch ausgebildeten Beatmungsschlauch gemäß dem Oberbegriff des Anspruchs 1. Ferner betrifft die Erfindung ein Verfahren zu dessen Herstellung gemäß Anspruch 15.

### Technologischer Hintergrund

Beheizbare Beatmungsschläuche mit einer flexiblen Schlauchwandung finden vielfach Verwendung. Insbesondere werden beheizbare Schläuche im Bereich der Intensiv-/Notfallmedizin, der Chirurgie/Anästhesie, der Beatmung/Entwöhnung sowie im Bereich der CPAP-, BIPAP- sowie Auto-PAP-Therapien eingesetzt. Die Schläuche besitzen eine möglichst dünne Schlauchwandung, um möglichst flexibel und leicht zu sein. Eine Beheizung der Beatmungsschläuche erfolgt, um eine Kondensation der sich im Schlauch ansammelnden Feuchte zu verhindern sowie Beatmungsgas in seiner Temperatur zu konditionieren. Die Beheizung der Schläuche erfolgt üblicherweise durch Heizdrähte in Form von Widerstandsdrähten, die entlang des Schlauchs verlaufen und Wärme in das Innere des Schlauchs abstrahlen. Üblicherweise werden zwei entlang des Schlauchs verlaufende Heizdrähte am einen Ende des Schlauchs miteinander kontaktiert und am anderen Ende des Schlauchs mit den Polen einer Spannungsquelle bzw. Stromquelle verbunden.

### Druckschriftlicher Stand der Technik

Ein Beatmungsschlauch gemäß dem Oberbegriff des Anspruchs 1 ist aus der US 6 367 510 bekannt. Bei diesem bekannten Beatmungsschlauch wird ein im Querschnitt kuppelförmiger Stabilisierungssteg mit einem im Querschnitt ovalen Hohlraum verwendet. Der Hohlraum und das Stegmaterial des Stabilisierungsstegs sind so geformt, dass durch den Hohlraum hindurch ein unter Druck stehendes Fluid entlang des Stabilisierungsstegs geleitet werden kann. Der Stabilisierungssteg wird im Bereich jeweils einer Überlappung der Lagen des den Schlauch bildenden Materials mit dem Schlauch verbunden. Es sind jeweils zwei Drähte pro Stabilisierungssteg vorgesehen, wobei es sich bei den Drähten insbesondere um Heizdrähte handeln kann.

Auch die US 2003/0183294 A1 offenbart einen länglichen Kunststoffschlauch, der einen kuppelförmigen, spiralförmig um den Kunststoffschlauch gewickelten Stabilisierungssteg aufweist. Der Schlauch enthält ein oder mehrere Lumen, die sich im Stabilisierungssteg von einem Ende des Schlauchs zum anderen Ende erstrecken. Außerdem kann der Schlauch einen oder mehrere elektrische Leiter enthalten, die sich ebenfalls spiralförmig von einem Ende zum anderen Ende des Schlauchs erstrecken.

Aus der DE 10 2009 009 790 B3 ist ein beheizbarer Schlauch bekannt, bei dem zumindest zwei aus Vollmaterial bestehende Verstärkungsrippen vorgesehen sind, wobei von jeder dieser Verstärkungsrippen ein einziger zur Beheizung des Schlauchs ausgelegter Heizdraht umgeben ist.

Die WO 2004/024429 A1 zeigt eine Konstruktion eines beheizbaren Schlauchs, bei dem jeweils ein Heizdrahtpaar innerhalb eines Umschlags des Wickelmaterials für den Schlauch untergebracht ist. Im Bereich des Umschlags findet sich gleichzeitig ein kuppelförmiger Stabilisierungssteg ohne Hohlraum.

Aus der DE 11 2013 005 798 T5 ist ein beheizbarer Beatmungsschlauch bekannt, der aus einem spiralförmig gewickelten Hohlkörper, der einen Teil des Lumens umgibt, sowie einem langgestreckten, Drähte aufweisenden Glied, welches zwischen den benachbarten Windungen des Hohlkörper positioniert wird, besteht. Die jeweiligen Drähte befinden sich somit jeweils im Randbereich des spiralförmig gewickelten Hohlkörpers.

Die US 2014/0202462 A1 betrifft medizinische Schläuche und Verfahren zur Herstellung medizinischer Schläuche. Der Schlauch kann eine Verbundstruktur sein, die aus zwei oder mehr verschiedenen Komponenten besteht, welche spiralförmig zur Bildung eines länglichen Schlauchs gewickelt sind. Zum Beispiel kann eine der Komponenten ein spiralförmig gewickelter länglicher Hohlkörper sein und die andere Komponente kann eine längliche strukturelle Komponente sein, die auch spiralförmig zwischen Wicklungen des spiralförmig gewickelten Hohlkörpers gewickelt ist. Der Schlauch muss jedoch nicht aus verschiedenen Komponenten bestehen. Zum Beispiel kann ein länglicher Hohlkörper, der aus einem einzigen Material gebildet ist, zur Bildung eines länglichen Schlauchs spiralförmig gewickelt sein. Der längliche Hohlkörper kann selbst im quer verlaufenden Querschnitt ein dünnes Wandteil und ein relativ dickeres oder steiferes Verstärkungsteil haben.

Die US 4,354,051 A betrifft einen elektrischen stromführenden flexiblen Schlauch, bei dem ein Streifen schraubenartig in Röhrenform gewunden ist. Der schraubenartig gewundene Streifen weist ineinandergreifende Zungen- und Nut-Randabschnitte auf, welche aneinandergrenzende Windungen verbinden. Auf dem Streifen befindet sich ein hohler Abschnitt, innerhalb dessen eine elektrische Leitung angeordnet ist.

### Aufgabe der vorliegenden Erfindung

Die Aufgabe der vorliegenden Erfindung besteht darin, einen gattungsgemäßen beheizbaren Beatmungsschlauch zu schaffen, welcher eine verbesserte Temperaturausbeute sowie eine verbesserte Temperaturregelung ermöglicht.

### Lösung der Aufgabe

Die vorstehende Aufgabe wird durch einen beheizbaren Beatmungsschlauch gemäß Anspruch 1 gelöst. Zweckmäßige Ausgestaltungen der vorliegenden Erfindung werden in den abhängigen Ansprüchen beansprucht.

Dadurch, dass der hohle rohrprofilartige Stabilisierungssteg im Bereich des Heizdrahts auf der dem Lumen zugewandten Seite des Stabilisierungsstegs eine gleichbleibende Wandstärke aufweist, also insbesondere die Innen- sowie Außenwand des Stabilisierungsstegs an dieser Stelle im Wesentlichen parallel zueinander verlaufen, wird eine gleichförmige thermische Isolierung zur Außenseite hin begründet, welche den Heizdraht oder die Heizdrähte zur Außenseite hin abschirmt, wodurch eine hervorragende thermische Isolierung des beheizbaren Beatmungsschlauchs nach außen erzielt werden kann. Daraus resultiert eine wesentlich verbesserte Temperaturausbeute nach innen als bisher. Ferner kann Atemgas wesentlich besser konditioniert bzw. die Konditionierung des Atemgases geregelt werden als bisher. Der erfindungsgemäße beheizbare Beatmungsschlauch kann bei Neonaten, Kindern sowie Erwachsenen zum Einsatz kommen.

Ferner kann der Stabilisierungssteg auf der dem Lumen abgewandten Seite des Stabilisierungsstegs ebenfalls eine gleichbleibende Wandstärke aufweisen. Auf diese Weise wird ein sich seitlich erstreckender Hohlraum mit hervorragenden thermischen Dämmeigenschaften im Inneren des Stabilisierungsstegs geschaffen.

Zweckmäßigerweise besitzen, in Querschnitt betrachtet, die Außenwand sowie die Innenwand des Stabilisierungsstegs, zumindest im Wesentlichen, bis auf den zwingenden Dimensionsunterschied die gleiche Form.

Insbesondere kann der Stabilisierungssteg, im Querschnitt betrachtet, rechteckige, quadratische oder teilkreisförmige Formen besitzen. Entsprechendes gilt insbesondere auch für Formen des im Stabilisierungssteg vorhandenen Hohlraums. Hierdurch kann eine optimale Abdeckung bzw. Isolierung erzielt werden.

Gemäß einer zweckmäßigen Ausgestaltung kann der Stabilisierungssteg mindestens einen Stützsteg aufweisen, der sich innerhalb des Lumens befindet und für eine mechanische Stützung der Form des Stabilisierungsstegs sorgt.

Beispielsweise kann der Stützsteg den Hohlraum, d.h. das Lumen, vorzugsweise mittig, durchsetzen und/oder eine gleichbleibende Dimension/Stärke aufweisen.

Dadurch, dass die Wandstärke des Innen- und Außenwand aufweisenden Schlauchs kleiner ist als die Wandstärke des Stabilisierungsstegs auf der dem Heizdraht zugewandten Seite wird eine optimale Wärmeabstrahlung des Heizdrahts auf das Innere des Schlauchs gewährleistet.

Vorzugsweise liegt die Wandstärke des die Innen- und Außenwand aufweisenden Schlauchs im Bereich von 0,14 mm bis 0,22 mm, vorzugsweise im Bereich von 0,16 mm bis 0,20 mm.

Ebenfalls vorzugsweise liegt die Wandstärke des Stabilisierungsstegs auf der dem Heizdraht zugewandten Seite im Bereich von 0,7 mm bis 1,2 mm, vorzugweise im Bereich von 0,5 mm bis 1,0 mm.

Hohlkörper und Stabilisierungssteg bestehen vorzugsweise aus dem gleichen Material, insbesondere aus einem extrudierbaren Material auf Kunststoffbasis. Vorzugsweise bestehen der Hohlkörper und/oder der Stabilisierungssteg aus PE oder PP oder aus einer Kombination von PE und PP. Besonders vorzugsweise können der Hohlkörper und/oder der Stabilisierungssteg auch aus Kunststoffen aus nachwachsenden oder bioabbaubaren Rohstoffen bestehen.

Erfindungsgemäß weist der Hohlkörper eine erste, dem Lumen zugewandte Schicht sowie eine zweite, dem Lumen abgewandte Schicht aufweist. Beide Schichten werden mit einem Überlappungsbereich oder auf Stoß gewickelt.

Des Weiteren betrifft die vorliegende Erfindung auch ein Verfahren zur Herstellung eines beheizbaren als Verbundschlauch ausgebildeten Beatmungsschlauchs gemäß den Verfahrensmerkmalen des Anspruchs 15. Zweckmäßigerweise wird der Hohlkörper, nachdem er gewickelt worden ist, vor dem Aufwickeln des Heizdrahts bzw. der Datenleitung auf die Außenfläche des Hohlkörpers gekühlt. Hierdurch wird sichergestellt, dass der Heizdraht bzw. die Datenleitung den Hohlkörper beim Aufwickeln nicht beschädigen kann. Zweckmäßigerweise wird eine jeweilige Anordnung bestehend aus mindestens zwei Heizdrähten sowie mindestens zwei Datenleitungen simultan als Doppelhelix aufgewickelt. Hierdurch kann das Herstellungsverfahren erheblich beschleunigt werden.

Darüber hinaus kann auch eine Anordnung bestehend aus mindestens zwei Stabilisierungsstegen simultan aufgewickelt werden. Auch hierdurch wird das Verfahren zur Herstellung des erfindungsgemäßen Beatmungsschlauchs beschleunigt.

Darüber hinaus ist, auch nebengeordnet beansprucht, vorgesehen, dass ein erster Stabilisierungssteg entlang der Außenwand des Hohlkörpers unter Bildung eines Wickelabstands spiralförmig gewickelt ist, entlang dessen mindestens zwei Heizdrähte verlaufen, und/oder ein zweiter Stabilisierungssteg entlang der Außenwand des Hohlkörpers unter Bildung eines Wickelabstands spiralförmig gewickelt ist, entlang dessen mindestens zwei Datenleitungen verlaufen. Daraus resultiert der Vorteil, dass insgesamt weniger Heizdrahtlänge und/oder Datenleitungslänge notwendig ist. Die erfindungsgemäße Konstruktion ermöglicht es zudem, zwei parallel verlaufende Heizdrähte wirksam nach außen hin thermisch zu isolieren.

Der Heizdraht kann hierbei an der Außenwand des Hohlkörpers aufliegen und im Übrigen von der Außenwand des Stabilisierungsstegs umschlossen sein. Der Stabilisierungssteg bettet den Heizdraht somit bis auf die Kontaktstelle mit der Außenwand des Hohlkörpers ein.

Alternativ kann der Heizdraht an der Außenwand der ersten Schicht des Hohlkörpers aufliegen und im Übrigen von der zweiten Schicht des Hohlkörpers umschlossen sein. Der Heizdraht ist hierbei in die Wand des Hohlkörpers eingebettet.

Vorzugsweise sind entlang des Hohlkörpers ein erster und zweiter Stabilisierungssteg spiralförmig um den Hohlraum d. h. Schlauch gewickelt. Sie bilden eine Doppelhelix.

Gemäß einer weiteren zweckmäßigen Ausgestaltung können entlang des ersten Stabilisierungsstegs mindestens zwei Heizdrähte verlaufen. Alternativ oder zusätzlich können entlang des zweiten Stabilisierungsstegs mindestens zwei Datenleitungen verlaufen.

Die vorliegende Erfindung betrifft ferner ein Verfahren zur Herstellung eines beheizbaren als Verbundschlauch ausgebildeten Beatmungsschlauchs mit folgenden Verfahrensschritten:
spiralförmiges Wickeln eines extrudierten flachen Bandes um einen Dorn, um einen Hohlkörper in Form eines länglichen Schlauchs mit Innen- und Außenwand, einer Längsachse und einem durch die Innenwand gebildeten Lumen, das sich entlang der Längsachse erstreckt, zu bilden,
spiralförmiges Aufwickeln einer Anordnung bestehend aus mindestens zwei parallel verlaufenden Heizdrähten auf die Außenfläche des Hohlkörpers,
spiralförmiges Aufwickeln eines extrudierten Stabilisierungsstegs auf die Außenfläche des Hohlkörpers im Bereich von mindestens zwei parallel verlaufenden benachbarten Heizdrähte, so dass nach dem Aufwickeln der mindestens zwei parallel verlaufenden Heizdrähten auf die Außenfläche des Hohlkörpers die Heizdrähte von dem Stabilisierungsstegs außenseitig umgeben sind, und/oder
spiralförmiges Aufwickeln einer Anordnung bestehend aus mindestens zwei parallel verlaufenden zwei Datenleitungen auf die Außenfläche des Hohlkörpers,
spiralförmiges Aufwickeln eines extrudierten Stabilisierungsstegs auf die Außenfläche des Hohlkörpers im Bereich von mindestens zwei parallel verlaufenden benachbarten Datenleitungen, so dass nach dem Aufwickeln der mindestens zwei Datenleitungen auf die Außenfläche des Hohlkörpers die Datenleitungen von dem Stabilisierungssteg außenseitig umgeben sind.

Vorzugsweise wird der Hohlkörper vor dem Aufwickeln der mindestens zwei Heizdrähte und/oder mindestens zwei Datenleitungen auf die Außenfläche des Hohlkörpers gekühlt. Hierdurch wird eine mechanische Beschädigung oder andere nachteilige Beeinträchtigung des Hohlkörpers durch den aufzuwickelnden Heizdraht oder der Datenleitung vermieden.

Nach dem Aufwickeln der jeweils mindestens zwei Heizdrähte und/oder Datenleitungen auf die Außenfläche des Hohlkörpers können die verbleibenden noch nicht abgedeckten Umfangsbereiche der Heizdrähte und/oder Datenleitungen in einfacher Weise in das Material des jeweilig aufzubringenden Stabilisierungsstegs eingebettet werden.

Erfindungsgemäß werden auch nach einem Aufwickeln der jeweils mindestens zwei Heizdrähte und/oder Datenleitungen auf die erste Schicht des Hohlkörpers die verbleibenden noch nicht abgedeckten Umfangsbereiche der Heizdrähte und/oder Datenleitungen anstatt in das Material des Stabilisierungsstegs in das Material der zweiten Schicht des Hohlkörpers eingebettet.

### Beschreibung der Erfindung anhand von Ausführungsbeispielen

Nachstehend werden verschiedene Ausführungsbeispiele der vorliegenden Erfindung anhand von Zeichnungen näher erläutert. Es zeigen:
- Fig. 1: ein Beispiel einer Gesamtanordnung, bei der ein Beatmungsschlauch gemäß der vorliegenden Erfindung zum Einsatz kommen kann;
- Fig. 2: eine erste Ausgestaltung eines erfindungsgemäßen Beatmungsschlauchs in Draufsicht (Fig. 2A), in einer Schnittdarstellung (Fig. 2B) sowie in einer vergrößerten Detailschnittdarstellung (Fig. 2C);
- Fig. 3: eine dritte Ausgestaltung des erfindungsgemäßen Beatmungsschlauchs in Draufsicht (Fig. 3A), in einer Schnittdarstellung (Fig. 3B), in einer vergrößerten Detailschnittdarstellung (Fig. 3C) sowie in einer vergrößerten Detailschnittdarstellung (Fig. 3D) eines Teils der Wandung des Hohlkörpers;
- Fig. 4: eine vierte Ausgestaltung des erfindungsgemäßen Beatmungsschlauchs in Draufsicht (Fig. 4A), in einer Schnittdarstellung (Fig. 4B) sowie in einer vergrößerten Detailschnittdarstellung (Fig. 4C);
- Fig. 5: eine vierte Ausgestaltung des erfindungsgemäßen Beatmungsschlauchs in Draufsicht (Fig. 5A), in einer Schnittdarstellung (Fig. 5B) sowie in einer vergrößerten Detailschnittdarstellung (Fig. 5C); sowie
- Fig. 6: eine stark vereinfachte schematische Darstellung der einzelnen Produktionsschritte (Fig. 6A - 6C) einer Ausgestaltung des Herstellungsverfahrens des erfindungsgemäßen Beatmungsschlauchs.

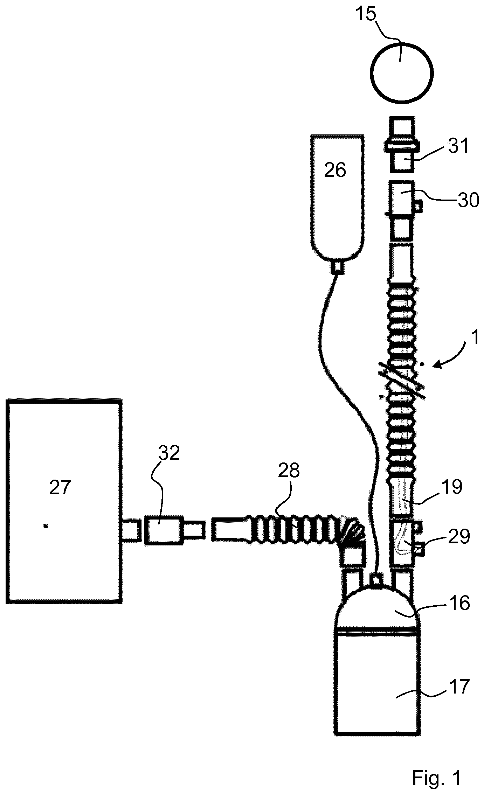

Fig. 1 illustriert ein Beispiel einer Gesamtanordnung, bei der ein Beatmungsschlauch gemäß der vorliegenden Erfindung zum Einsatz kommen kann. Der Beatmungsschlauch 1 befindet sich z. B. zwischen einem Befeuchter 16 mit Befeuchterkammer und einer am Patienten vorgesehenen Beatmungshilfe z. B. Beatmungsmaske 15. In der Befeuchterkammer des Befeuchters 16 wird Beatmungsluft konditioniert d. h. über eine Zufuhr von sterilem Wasser aus einem Reservoir 26 auf einen bestimmten Feuchtegrad und eine bestimmte Temperatur gebracht und dem Patienten über den Beatmungsschlauch 1 zugeführt. Der Beatmungsschlauch 1 wird hierbei mittels eines Heizdrahts beheizt, um zu vermeiden, dass Feuchtigkeit an der Innenwandung des Beatmungsschlauchs 1 kondensiert. Die Steuerung der Konditionierung d. h. des Feuchtegrads, der Temperatur sowie der Heizleistung erfolgt über ein entsprechendes Steuergerät 17 des Befeuchters 16. Der Befeuchter 16 steht über einen Schlauch 28 und eine Muffe 32 mit einem Ventilator 27 in Verbindung. Der Ventilator 27 verursacht den notwendigen Flow von konditioniertem Beatmungsgas vom Befeuchter 16 hin zum Patienten.

Der Beatmungsschlauch 1 besitzt an jedem Ende ein Anschlussstück 18, 19, welches vorzugsweise integraler Bestandteil des Beatmungsschlauchs 1 ist und jeweils dazu dient, eine Steckverbindung mit dem betreffenden Anschlussteil zu ermöglichen. In der in Fig. 1 dargestellten beispielhaften Anordnung wird das Anschlussstück 18 beispielsweise über einen Konnektor 30 mit einer Sondenbohrung mit einem sogenannten Leckageventil 31 sowie das gegenüberliegende Anschlussstück 19 am Beatmungsschlauch 1 beispielsweise über einen Konnektor 29 mit einem elektrischen Stecker für den Heizdraht sowie Sondenbohrung mit einem Anschluss der Befeuchterkammer 16 verbunden. Die Anordnung gemäß Fig. 1 stellt lediglich ein Anwendungsbeispiel dar. Derartige Beatmungsschläuche werden im Bereich der Intensiv-/Notfallmedizin, der Chirurgie/Anästhesie, der Beatmung/Entwöhnung sowie im Bereich der CPAP-, BIPAPsowie Auto-PAP-Therapien eingesetzt. Insbesondere werden solche Beatmungsschläuche auch bei der Beatmung von Frühgeborenen eingesetzt. Die unreife Lunge produziert bei Frühgeborenen nur in geringem Maße Surfactant. Dadurch kollabieren Lungenbläschen, die dann am Gasaustausch nicht teilnehmen können. Sauerstoffmangel und Atemnot sind die Folgen. In den zusammengefallenen Lungenbläschen sammeln sich Blutproteine an, und es entstehen hyaline Membranen. Zur Behandlung wird das Frühgeborene intubiert und künstlich beatmet. Gerade für diesen Einsatzzweck ist eine sehr genaue Konditionierung und Steuerung des Atemgases von ganz besonderer Bedeutung. Der erfindungsgemäße beheizbare Beatmungsschlauch kann bei Neonaten (Schlauchdurchmesser 10 mm), Kindern (Schlauchdurchmesser 15 mm) sowie Erwachsenen (Schlauchdurchmesser 22 mm) zum Einsatz kommen.

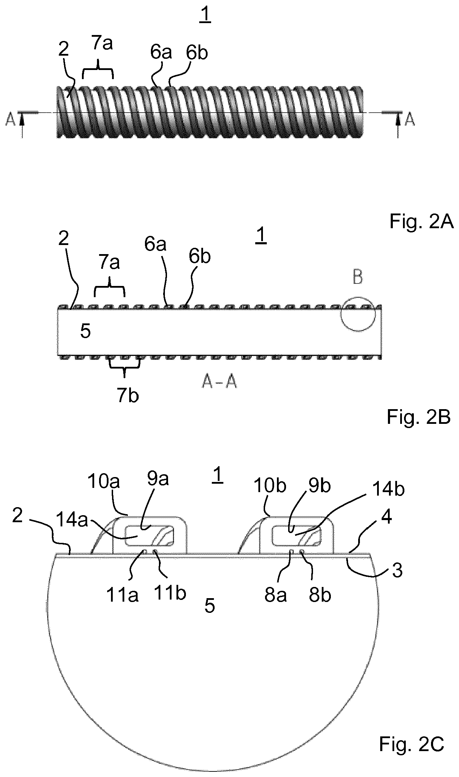

Fig. 2A zeigt einen Teilbereich eines beheizbaren Beatmungsschlauchs, wie er in Fig. 1 dargestellt ist, gemäß einer ersten Ausgestaltung der Erfindung in Draufsicht. Der Beatmungsschlauch 1 umfasst einen Schlauch in Form eines Hohlkörpers 2, der helixförmig aus einzelnen (in Fig. 2A nicht dargestellten) im Randbereich auf Stoß aneinander liegenden oder überlappenden Bändern gewickelt ist. Allein dieser Hohlkörper 2 legt das Lumen 5 des Beatmungsschlauchs 1 fest. Das Lumen 5 des Beatmungsschlauchs 1 besitzt eine entlang des Beatmungsschlauchs 1 gleichbleibende Querschnittsfläche.

Die Wandstärke des Hohlkörpers 2 bzw. Schlauchs ist vergleichsweise gering. Sie liegt insbesondere im Bereich von 0,14 mm bis 0,22 mm, vorzugsweise im Bereich von 0,16 mm bis 0,20 mm. Bei der in Fig. 2 gezeigten Ausgestaltung umfasst der Hohlkörper 2 nur eine Schicht.

Der Hohlkörper 2 besteht aus einem extrudierbaren thermoplastischen Kunststoffmaterial, vorzugsweise aus PE oder PP oder aus einer Kombination von PE und PP. Besonders vorzugsweise kann der Hohlkörper auch aus Kunststoffen aus nachwachsenden oder bioabbaubaren Rohstoffen bestehen.

Auf der Außenseite des Hohlkörpers 2 befinden sich zwei in Form einer Doppelhelix spiralförmig aufgebrachte Stabilisierungsstege 6a, 6b, die in einem jeweils festen Abstand 7a bzw. 7b auf die Außenwand 4 des Hohlkörpers 2 aufgewickelt sind. Bei der Ausgestaltung gemäß Fig. 2A sind die Abstände 7a und 7b identisch. Der jeweilige Stabilisierungssteg 6a, 6b umfasst jeweils eine Sohle, eine Decke an der Oberseite sowie seitlich je eine Schulter, die Sohle und Decke miteinander verbinden. Der unmittelbare Abstand zweier angrenzender Stabilisierungsstege 6a, 6b entspricht zumindest in etwa der Stegbreite des jeweiligen Stabilisierungsstegs 6a, 6b. Die Stabilisierungsstege 6a, 6b geben dem Beatmungsschlauch 1 die erforderliche mechanische Stabilität sowie Druckfestigkeit. Darüber hinaus dienen sie dazu, Leitungen, die für Konditionierung des Atemgases in dem Beatmungsschlauch 1 notwendig sind, abzudecken. Wie aus den Fig. 1A und 1B ersichtlich, können die beiden Stabilisierungsstege 6a, 6b eine gleiche Form und/oder Dimensionierung aufweisen.

Die betreffenden Leitungen und deren Anordnung sind aus Fig. 2C gut erkennbar. Gezeigt sind jeweils ein Stabilisierungssteg 6a, im Bereich dessen sich z. B. zwei Datenleitungen 11a, 11b befinden, sowie ein weiterer Stabilisierungssteg 6b, im Bereich dessen sich z. B. zwei Heizdrähte 8a, 8b befinden.

Wie aus Fig. 2C weiter hervorgeht, weist der Stabilisierungssteg 6b im Bereich des Heizdrahts 8a, 8b auf der dem Lumen 5 zugewandten Seite des Stabilisierungsstegs 6b eine gleichbleibende Wandstärke auf, d. h. er umfasst in diesem Bereich demzufolge jeweils eine Innenwand 9a, 9b sowie Außenwand 10a, 10b. Wie aus Fig. 2C ebenfalls deutlich hervorgeht, verläuft die Innenwand 9a, 9b sowie Außenwand 10a, 10b im Bereich des Heizdrahts 8a, 8b bzw. der Datenleitung 11a, 11b zumindest im Wesentlichen parallel zueinander. Hierdurch wird erreicht, dass die beiden Heizdrähte 8a, 8b bzw. Datenleitungen 11a, 11b zur Außenseite hin über eine erhebliche Breite durch eine Luftkammer bzw. einen Hohlraum zur Außenseite hin abgedeckt sind, wodurch sich eine besonders gute Isolierung zur Außenseite hin einstellt.

Die Anordnung der Datenleitungen 11a, 11b bzw. Heizdrähte 8a, 8b befindet sich vorzugsweise mittig in Bezug auf die Form des jeweiligen Stabilisierungsstegs 6a, 6b.

Der jeweilige Stabilisierungssteg 6a, 6b der Ausgestaltung nach Fig. 2C besitzt z. B. eine rechteckige Form. Vorzugsweise entspricht, bis auf die Reduzierung der Dimension, die Außenform des Stabilisierungsstegs 6a, 6b, zumindest im Wesentlichen, der Form des zugehörigen Hohlraums 14a, 14b. Demzufolge besitzt jeder Stabilisierungssteg 6a, 6b bereichsweise mehr oder weniger gleichbleibende, umlaufende Wandbereiche.

Der jeweilige Stabilisierungssteg 6a, 6b besteht ebenfalls aus einem extrudierbaren Kunststoffmaterial, vorzugsweise aus dem gleichen Material wie der Hohlkörper 2. Insbesondere wird hierfür als Material PE und/oder PP oder ein Kunststoff aus nachwachsenden oder bioabbaubaren Rohstoffen verwendet.

Bei der Ausgestaltung nach Fig. 2C besitzt der jeweilige Stabilisierungssteg 6a, 6b im Querschnitt betrachtet rechteckige Form. Er kann jedoch genauso eine quadratische Form besitzen.

Der Abstand zweier benachbarter Stabilisierungsstege 6a, 6b zueinander entspricht in etwa der Breite eines Stabilisierungsstegs.

Die Wandstärke des die Innenwand 3 und Außenwand 4 aufweisenden Schlauchs bzw. Hohlkörpers 2 ist vorzugsweise kleiner als die Wandstärke des Stabilisierungsstegs 6a, 6b auf der dem Heizdraht 8a, 8b und/oder der Datenleitung 11a, 11b bzw. dem Lumen 5 zugewandten Seite. Hierdurch wird ein optimierter Wärmeaustausch zwischen dem Inneren des Schlauchs und dem Heizdraht erreicht.

Ferner liegt bei dieser Ausgestaltung der Heizdraht 8a, 8b an der Außenwand 4 des Hohlkörpers 2 auf. Im Übrigen ist der Heizdraht 8a, 8b von der Außenwand 10a, 10b des Stabilisierungsstegs 6a, 6b umschlossen, d. h. der Rest ist in die Wandung des Stabilisierungsstegs 6a, 6b eingebettet.

Die Wandstärke des die Innen- 3 und Außenwand 4 aufweisenden Schlauchs liegt im Bereich von 0,14 mm bis 0,22 mm, vorzugsweise im Bereich von 0,16 mm bis 0,20 mm.

Die Wandstärke des Stabilisierungsstegs 6a, 6b auf der dem Heizdraht 8a, 8b zugewandten Seite liegt im Bereich von 0,7 mm bis 1,2 mm, vorzugsweise im Bereich von 0,5 mm bis 1,0.

Hohlkörper 2 und der Stabilisierungssteg 6a, 6b bestehen vorzugsweise aus dem gleichen Material, z. B. aus PE und/oder PP oder einem Kunststoff aus nachwachsenden oder bioabbaubaren Rohstoffen.

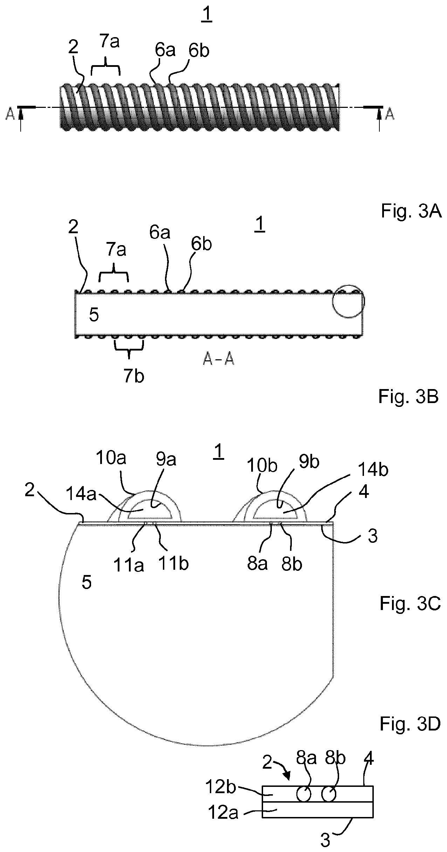

Bei der in den Fig. 3A - 3C dargestellten Ausführungsform besitzen die beiden Stabilisierungsstege 6a, 6b eine halbkreisförmige oder kreisabschnittförmige Form.

Auch hier weist der Stabilisierungssteg 6b im Bereich des Heizdrahts 8a, 8b auf der dem Lumen 5 zugewandten Seite des Stabilisierungsstegs 6b eine gleichbleibende Wandstärke auf. Er umfasst jeweils eine Innenwand 9a, 9b sowie Außenwand 10a, 10b, deren Formverlauf zumindest im Wesentlichen gleich ist. Wie aus Fig. 3C ebenfalls deutlich hervorgeht, verläuft die Innenwand 9a, 9b sowie Außenwand 10a, 10b im Bereich des Heizdrahts 8a, 8b bzw. der Datenleitung 11a, 11b zumindest im Wesentlichen parallel zueinander. Auch hier liegen die beiden Heizdrähte 8a, 8b und/oder Datenleitungen 11a, 11b jeweils mittig zum zugehörigen Stabilisierungssteg 6a, 6b.

Die Einbettung der beiden Heizdrähte 8a, 8b und/oder Datenleitungen 11a, 11b unterscheidet sich von der Ausgestaltung nach Fig. 2. darin, dass bei dieser Variante nach Fig. 3C der Hohlkörper 2 zwei Schichten umfasst d. h. aus einer ersten inneren Schicht 12a sowie einer zweiten äußeren Schicht 12b aufgebaut ist, vgl. den vergrößerten Ausschnitt des Bereichs der beiden Heizdrähte gemäß Fig. 3D. Die erste innere Schicht 12a wird zuerst gewickelt, danach werden die beiden Heizdrähte 8a, 8b und die Datenleitungen 11a, 11b aufgebracht. Dann wird die zweite äußere Schicht 12b gewickelt. Die beiden Schichten 12a, 12b können jeweils mit einem Überlappungsbereich oder auf Stoß gewickelt werden. Die beiden Heizdrähte 8a, 8b und/oder Datenleitungen 11a, 11b liegen auf der Außenseite der ersten Schicht 12a auf und sind in die zweite Schicht 12b eingebettet.

Die dem Lumen 5 zugewandte Wand des jeweiligen Stabilisierungsstegs 6a, 6b liegt außen an der zweiten Schicht 12b des Hohlkörpers 2 also an dessen Außenwand 4 auf. Die dem Lumen 5 zugewandte Wand des jeweiligen Stabilisierungsstegs 6a, 6b weist eine Stärke auf, die größer ist als die Stärke der beiden Schichten 12a, 12b zusammen.

Bei der in Fig. 3C gezeigten Ausführungsform hat der Stabilisierungssteg 6a, 6b im Querschnitt betrachtet eine halbkreisförmige oder kreisabschnittförmige Form. Die dem Lumen 5 zugewandte Wand des jeweiligen Stabilisierungsstegs 6a, 6b besitzt eine gleichbleibende Wandstärke, ebenso wie die dem Lumen 5 abgewandte Wand des jeweiligen Stabilisierungsstegs 6a, 6b. Im Inneren befindet sich ein bis auf die Dimensionsreduzierung der äußeren Form entsprechend geformter Hohlraum 14a, 14b.

Die dem Lumen 5 zugewandte Wandstärke kann kleiner oder gleich der dem Lumen 5 abgewandte Wandstärke des jeweiligen Stabilisierungsstegs 6a, 6b sein.

Der Abstand zweier benachbarter Stabilisierungsstege 6a, 6b zueinander entspricht in etwa der Breite eines Stabilisierungsstegs. Hierdurch ergibt sich ein optimales Verhältnis von Flexibilität sowie mechanischer Stabilität. Im Übrigen wird auf die Angaben und Dimensionierungen der vorher beschriebenen Ausgestaltung verwiesen.

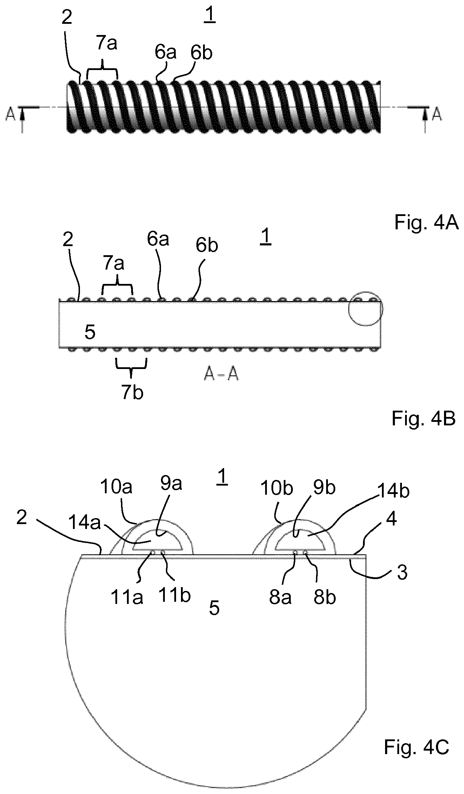

Die Fig. 4A-4C beschreiben eine Ausgestaltung des erfindungsgemäßen beheizbaren Beatmungsschlauchs, bei der die Heizdrähte 8a, 8b sowie Datenleitungen 11a, 11b nicht in die Wandung des Hohlkörpers 2, sondern wie bei der Ausgestaltung gemäß Fig. 2A-2C in die dem Lumen 5 zugeordnete Wandung des jeweiligen Stabilisierungsstegs 6a, 6b eingebettet sind. Der Hohlraum 2 ist bei der in Fig. 4C dargestellten Ausgestaltung lediglich einschichtig aufgebaut.

Die Querschnittsform der Stabilisierungsstege 6, 6b entsprechen jedoch im Übrigen der Ausgestaltung gemäß Fig. 3. Im Übrigen wird auf die Angaben und Dimensionierungen der vorher beschriebenen Ausgestaltungen verwiesen.

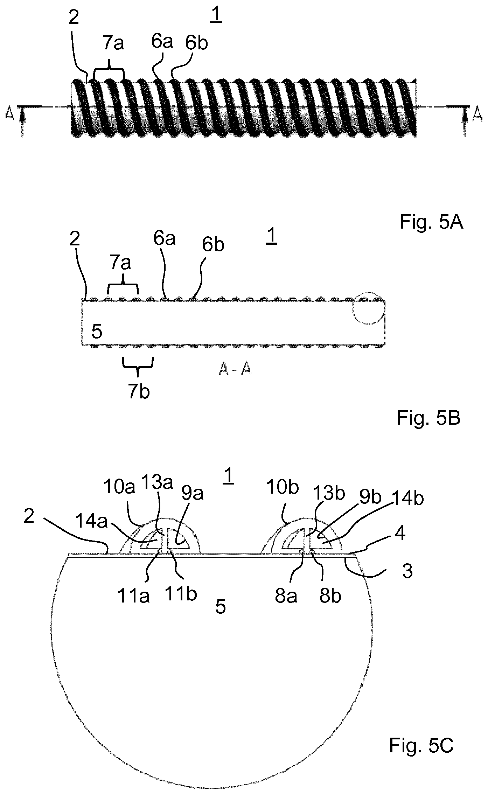

In den Fig. 5A-5C wird eine weitere Ausgestaltung des erfindungsgemäßen beheizbaren Beatmungsschlauchs 1 gezeigt. Bei dieser Ausgestaltung ist z.B. ein Stützsteg 13a, 13b innerhalb des Stabilisierungsstegs 6a, 6b vorgesehen. Der Stützsteg 13a, 13b verläuft beispielsweise senkrecht zur dem Lumen 5 zugeordneten Wand des betreffenden Stabilisierungsstegs 6a, 6b und teilt den betreffenden Hohlraum 14a, 14b in zwei, vorzugsweise im Querschnitt identische, Teilhohlräume auf. Auch bei dieser Ausgestaltung hat der jeweilige Stabilisierungssteg 6a und/oder 6b im Bereich des Heizdrahts 8a, 8b und/oder der Datenleitung 11a, 11b auf der dem Lumen 5 zugewandten Seite des betreffenden Stabilisierungsstegs 6a 6b und/oder auf der dem Lumen 5 abgewandten Seite des betreffenden Stabilisierungsstegs 6a, 6b eine gleichbleibende Wandstärke. Die vorbeschriebene Ausgestaltung gewährleistet bedarfsweise eine zusätzliche mechanische Stabilität der Stabilisierungsstege 6a, 6b, gewährt jedoch aufgrund der Ausgestaltung der Stabilisierungsstege 6a, 6b ebenfalls eine sehr gute thermischer Dämmung der Heizdrähte 8a, 8b zur Außenseite hin.

Bei der Ausgestaltung gemäß den Fig. 5A-5C sind die Heizdrähte 8a, 8b und/oder die Datenleitungen 11a, 11b ebenfalls in die Wand des jeweiligen Stabilisierungsstegs eingebettet. In dieser Hinsicht entspricht diese Ausgestaltung der Ausgestaltung gemäß den Fig. 2A-2C. Im Übrigen entspricht die Ausgestaltung den vorbeschriebenen Ausgestaltungen.

Es sei darauf hingewiesen, dass die Einbettung der Heizdrähte 8a, 8b und/oder Datenleitungen 11a, 11b der beschriebenen Ausführungsbeispiele auch in der jeweils alternativen Ausgestaltung erfolgen kann.

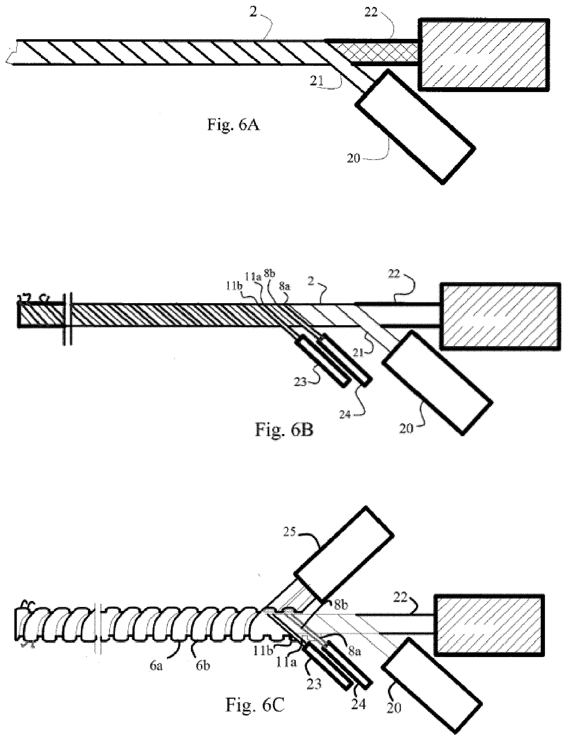

In den Fig. 6A-6C werden die einzelnen Produktionsschritte einer Ausgestaltung eines Herstellungsverfahrens zur Herstellung des erfindungsgemäßen beheizbaren Beatmungsschlauchs gezeigt, bei der z. B. gemäß Fig. 2A-2C die Heizdrähte 8a, 8b und/oder die Datenleitungen 11a, 11b in die Wand des jeweiligen Stabilisierungsstegs 6a, 6b eingebettet sind.

Über einen ersten Extruder 20 wird zunächst eine Materialbahn 21 aus einem geeigneten Kunststoffmaterial, z.B. PP oder PE, erzeugt. Die Materialbahn 21 des Extruders 20 ist in einer schrägen Winkelstellung zu einem Drehdorn 22 orientiert. Durch Kontaktierung der Materialbahn 21 mit dem rotierenden Drehdorn 22 wird die Materialbahn 21 sukzessive auf den Drehdorn 22 aufgewickelt und bildet eine helixförmige Wicklung. Bei der in Fig. 6A dargestellten Verfahrensweise wird die Materialbahn 21 auf dem Drehdorn 22 "auf Stoß" gewickelt, d.h. bildet keinen Überlappungsbereich aus. Alternativ kann die Materialbahn 21 jedoch auch derart gewickelt werden, dass zwischen den benachbarten Materialbahnen 21 ein gewisser Überlappungsbereich entsteht.

Nach Aufwickeln der Materialbahn 21 auf den Drehdorn 22 wird der hierdurch entstehende Hohlkörper gekühlt, sodass sich das Material der Materialbahn 21 etwas verfestigt. Im Anschluss daran wird über eine Drahtzuführeinrichtung 23 bzw. 24 je ein Heizdrahtpaar 8a, 8b sowie ein Datenleitungspaar 11a, 11b, vorzugsweise simultan, auf die Oberfläche des durch die Materialbahn 21 gebildeten Hohlkörpers 2 aufgewickelt. Die Heizdrähte 8a, 8b sowie Datenleitungen 11a, 11b liegen jeweils paarweise vor und sind jeweils paarweise zwischen den Stoßstellen der Materialbahn 21 positioniert.

Nachdem die Heizdrähte 8a, 8b sowie Datenleitungen 11a, 11b aufgewickelt worden sind, wird über einen zweiten Extruder 25 der jeweilige Stabilisierungssteg 6a, 6b so auf der Oberseite des Schlauchs bzw. Hohlraums 2 aufgebracht, dass das jeweilige Paar aus Heizdrähten 8a, 8b bzw. Datenleitungen 11a, 11b sich mittig unterhalb des zugehörigen Stabilisierungsstegs 6a, 6b befinden. Das Material des Stabilisierungsstegs 6a, 6b ist hierbei noch in fließfähigem Zustand, so dass die freien Oberflächenbereiche der Heizdrähten 8a, 8b bzw. Datenleitungen 11a, 11b umschlossen werden und hierdurch ein Einschluss derselben durch die Wandung des jeweiligen Stabilisierungsstegs 6a, 6b. erfolgt.

Die Extrusion der beiden Stabilisierungsstege 6a, 6b erfolgt vorzugsweise simultan. Hierdurch wird eine Doppelhelix zweier Stabilisierungsstege 6a, 6b geschaffen, die die jeweiligen Heizdrähte 8a, 8b sowie Datenleitungen 11a, 11b zur Außenseite hin thermisch isolieren. Hierdurch wird ein Wärmeverlust nach außen verhindert. Durch die Konstruktion wird gleichzeitig ein Wärmetransfer vom Heizdraht 8a, 8b zum Lumen 5 des Schlauchs 2 erleichtert.

Alternativ kann das Verfahren auch derart erfolgen, dass nach dem Aufbringen der Heizdrähte 8a, 8b sowie Datenleitungen 11a, 11b (Fig. 6B) in einem weiteren Verfahrensschritt entsprechend dem Verfahrensschritt gemäß Fig. 6A mit Material einer weiteren Materialbahn eine weitere Schicht aufgebracht wird, um die Heizdrähte 8a, 8b und/oder Datenleitungen 11a, 11b entsprechend der Ausgestaltung nach Fig. 3A bis 3D in die weitere Schicht des Holkörpers 2 einzubetten. Danach erfolgt das Aufbringen der Stabilisierungsstege 6a, 6b auf die freiliegenden Heizdrähte 8a, 8b und/oder Datenleitungen 11a, 11b. Danach werden zur Fertigstellung des beheizbaren Beatmungsschlauchs 1 Endmuffen nachträglich angespritzt. Steckverbindungen für den Heizdraht 8a, 8b sowie die Temperaturmessung werden mechanisch kodiert.

Obwohl die Erfindung beispielhaft und unter Bezugnahme auf mögliche Ausführungsbeispiele dieser beschrieben wurde, sollte verstanden werden, dass Modifikationen oder Verbesserungen an diesen vorgenommen werden können, ohne den Umfang und Rahmen der Erfindung zu verlassen und ohne ihre dazugehörigen Vorteile abzumildern. Darüber hinaus wird ausdrücklich darauf hingewiesen, dass auch Unterkombinationen sowie Einzelmerkmale bzw. Merkmalsgruppen der beschriebenen Ausführungsformen für sich gesehen als erfindungswesentlich angesehen werden.

### BEZUGSZEICHENLISTE

- 1: Beatmungsschlauch
- 2: Hohlkörper
- 3: Innenwand
- 4: Außenwand
- 5: Lumen
- 6a: Stabilisierungssteg
- 6b: Stabilisierungssteg
- 7a: Abstand
- 7b: Abstand
- 8a: Heizdraht
- 8b: Heizdraht
- 9a: Innenwand
- 9b: Innenwand
- 10a: Außenwand
- 10b: Außenwand
- 11a: Datenleitung
- 11b: Datenleitung
- 12a: erste Schicht des Hohlkörpers
- 12b: zweite Schicht des Hohlkörpers
- 13a: Stützsteg
- 13b: Stützsteg
- 14a: Hohlraum
- 14b: Hohlraum
- 15: Beatmungsmaske
- 16: Befeuchterkammer
- 17: Steuergerät
- 18: Anschlussstück
- 19: Anschlussstück
- 20: erster Extruder
- 21: Materialbahn
- 22: Drehdorn
- 23: Drahtzuführeinrichtung
- 24: Drahtzuführeinrichtung
- 25: zweiter Extruder
- 26: Reservoir
- 27: Ventilator
- 28: Schlauch
- 29: Konnektor
- 30: Konnektor
- 31: Leckageventil
- 32: Anschlussmuffe

## Patentansprüche

1. Beheizbarer als Verbundschlauch ausgebildeter Beatmungsschlauch (1) umfassend
einen Hohlkörper (2), der spiralförmig gewickelt ist, um einen länglichen Schlauch mit Innen- (3) und Außenwand (4), einer Längsachse und einem durch die Innenwand (3) gebildeten Lumen (5), das sich entlang der Längsachse erstreckt, zu bilden,
mindestens einen, mindestens einen Hohlraum (14a, 14b) aufweisenden Stabilisierungssteg (6a, 6b) mit einer Innen- (9a, 9b) sowie Außenwand (10a, 10b), der entlang der Außenwand (4) des Hohlkörpers (2) unter Bildung eines Wickelabstands (7a, 7b) spiralförmig gewickelt und mit dem Hohlkörper (2) verbunden ist, sowie
mindestens einen Heizdraht (8a, 8b), der von einem Stabilisierungssteg (6b) zur Außenseite hin abgedeckt ist, wobei
der Stabilisierungssteg (6b) im Bereich des Heizdrahts (8a, 8b) auf der dem Lumen (5) zugewandten Seite des Stabilisierungsstegs (6b) eine gleichbleibende Wandstärke aufweist,
**dadurch gekennzeichnet, dass**
der Hohlkörper (2) eine erste, dem Lumen (5) zugewandte Schicht (12a) sowie eine zweite, dem Lumen abgewandte Schicht (12b) aufweist.

2. Beatmungsschlauch nach Anspruch 1, **dadurch gekennzeichnet, dass** der Stabilisierungssteg (6b) auf der dem Lumen (5) abgewandten Seite des Stabilisierungsstegs (6b) eine gleichbleibende Wandstärke aufweist.

3. Beatmungsschlauch nach den vorhergehenden Ansprüchen, **dadurch gekennzeichnet, dass** der Stabilisierungssteg (6a, 6b), im Querschnitt betrachtet, rechteckige, quadratische oder teilkreisförmige Form besitzt.

4. Beatmungsschlauch nach den vorhergehenden Ansprüchen, **dadurch gekennzeichnet, dass** der Stabilisierungssteg (6a, 6b) mindestens einen Stützsteg (13a, 13b) aufweist.

5. Beatmungsschlauch nach Anspruch 4, **dadurch gekennzeichnet, dass** der Stützsteg (13a, 13b) den Hohlraum (14a, 14b), vorzugsweise mittig, durchsetzt und/oder der Stützsteg (13a, 13b) eine gleichbleibende Dimension aufweist.

6. Beatmungsschlauch nach den vorhergehenden Ansprüchen, **dadurch gekennzeichnet, dass** die Wandstärke des die Innen- (3) und Außenwand (4) aufweisenden Schlauchs kleiner ist als die Wandstärke des Stabilisierungsstegs (6a, 6b) auf der dem Lumen (5) zugewandten Seite Seite.

7. Beatmungsschlauch nach den vorhergehenden Ansprüchen, **dadurch gekennzeichnet, dass** Wandstärke des die Innen- (3) und Außenwand (4) aufweisenden Schlauchs im Bereich von 0,14 mm bis 0,22 mm, vorzugsweise im Bereich von 0,16 mm bis 0,20 mm liegt.

8. Beatmungsschlauch nach den vorhergehenden Ansprüchen, **dadurch gekennzeichnet, dass** die Wandstärke des Stabilisierungsstegs (6a, 6b) auf der dem Heizdraht (8a, 8b) zugewandten Seite im Bereich von 0,7 mm bis 1,2 mm, vorzugsweise im Bereich von 0,5 mm bis 1,0mm.

9. Beatmungsschlauch nach den vorhergehenden Ansprüchen, **dadurch gekennzeichnet, dass** der Hohlkörper (2) und der Stabilisierungssteg (6a, 6b) aus dem gleichen Material bestehen.

10. Beatmungsschlauch nach den vorhergehenden Ansprüchen, **dadurch gekennzeichnet, dass** das Material des Hohlkörpers (2) jeweils spiralförmig mit einem Überlappungsbereich oder auf Stoß gewickelt ist.

11. Beatmungsschlauch nach den vorhergehenden Ansprüchen, **dadurch gekennzeichnet, dass** der Heizdraht (8a, 8b) an der Außenwand (4) des Hohlkörpers (2) aufliegt und im Übrigen von der Außenwand (10a, 10b) des Stabilisierungsstegs (6a, 6b) umschlossen ist.

12. Beatmungsschlauch nach Anspruch 1 oder 10, **dadurch gekennzeichnet, dass** der Heizdraht (8a, 8b) an der Außenwand der ersten Schicht (12a) des Hohlkörpers (2) aufliegt und im Übrigen von der zweiten Schicht (12b) des Hohlkörpers (2) umschlossen ist.

13. Beatmungsschlauch nach den vorhergehenden Ansprüchen, **dadurch gekennzeichnet, dass** ein erster Stabilisierungssteg (6a) entlang der Außenwand (4) des Hohlkörpers (2) unter Bildung eines Wickelabstands (7a) spiralförmig gewickelt ist, entlang dessen mindestens zwei Heizdrähte (8a, 8b) verlaufen, und/oder
ein zweiter Stabilisierungssteg (6b) entlang der Außenwand (4) des Hohlkörpers (2) unter Bildung eines Wickelabstands (7b) spiralförmig gewickelt ist, entlang dessen mindestens zwei Datenleitungen (11a, 11b) verlaufen.

14. Beatmungsschlauch nach den vorhergehenden Ansprüchen, **dadurch gekennzeichnet, dass** ein der Abstand zweier benachbarter Stabilisierungsstege (6a, 6b) zueinander zumindest in etwa der Breite eines Stabilisierungsstegs entspricht.

15. Verfahren zur Herstellung eines beheizbaren als Verbundschlauch ausgebildeten Beatmungsschlauchs mit **folgenden Verfahrensschritten:**
spiralförmiges Wickeln eines extrudierten flachen Bandes um einen Dorn (14), um einen Hohlkörper (2) in Form eines länglichen Schlauchs mit Innen- (3) und Außenwand (4), einer Längsachse und einem durch die Innenwand (3) gebildeten Lumen (5), das sich entlang der Längsachse erstreckt, zu bilden, wobei der Hohlkörper (2) eine erste, dem Lumen (5) zugewandte Schicht (12a) sowie eine zweite, dem Lumen abgewandte Schicht (12b) aufweist,
spiralförmiges Aufwickeln einer Anordnung bestehend aus mindestens zwei parallel verlaufenden Heizdrähten (8a, 8b) auf die Außenfläche des Hohlkörpers (2),
spiralförmiges Aufwickeln eines extrudierten Stabilisierungsstegs (6b) auf die Außenfläche des Hohlkörpers (2) im Bereich von mindestens zwei parallel verlaufenden benachbarten Heizdrähte (8a, 8b), so dass nach dem Aufwickeln der mindestens zwei parallel verlaufenden Heizdrähte (8a, 8b) auf die Außenfläche des Hohlkörpers (2) die Heizdrähte (8a, 8b) von dem Stabilisierungssteg (6b) außenseitig umgeben sind, und/oder
spiralförmiges Aufwickeln einer Anordnung bestehend aus mindestens zwei parallel verlaufenden zwei Datenleitungen (11a, 11b) auf die Außenfläche des Hohlkörpers (2),
spiralförmiges Aufwickeln eines extrudierten Stabilisierungsstegs (6a) auf die Außenfläche des Hohlkörpers (2) im Bereich von mindestens zwei parallel verlaufenden benachbarten Datenleitungen (11a, 11b), so dass nach dem Aufwickeln der mindestens zwei Datenleitungen (11a, 11b) auf die Außenfläche des Hohlkörpers (2) die Datenleitungen (11a, 11b) von dem Stabilisierungssteg (6b) außenseitig umgeben sind, und
nach dem Aufwickeln der jeweils mindestens zwei Heizdrähte (8a, 8b) und/oder Datenleitungen (11a, 11b) auf die erste Schicht (12a) des Hohlkörpers (2) die verbleibenden noch nicht abgedeckten Umfangsbereiche der Heizdrähte (8a, 8b) und/oder Datenleitungen (11a, 11b) in das Material der zweiten Schicht (12b) des Hohlkörpers (2) eingebettet werden.

16. Verfahren nach Anspruch 15, **dadurch gekennzeichnet, dass** der Hohlkörper (2) vor dem Aufwickeln der mindestens zwei Heizdrähte (8a, 8b) und/oder mindestens zwei Datenleitungen (11a, 11b) auf die Außenfläche des Hohlkörpers (2) gekühlt wird.

17. Verfahren nach Anspruch 15 oder 16, **dadurch gekennzeichnet, dass** nach dem Aufwickeln der jeweils mindestens zwei Heizdrähte (8a, 8b) und/oder Datenleitungen (11a, 11b) auf die Außenfläche des Hohlkörpers (2) die verbleibenden noch nicht abgedeckten Umfangsbereiche der Heizdrähte (8a, 8b) und/oder Datenleitungen (11a, 11b) in das Material des Stabilisierungsstegs (6a, 6b) eingebettet werden.

## Claims

1. Heatable breathing tube (1) designed as a composite tube, comprising
a hollow body (2) which is wound in a spiral shape in order to form an elongate tube with inner wall (3) and outer wall (4), a longitudinal axis, and a lumen (5) formed by the inner wall (3) and extending along the longitudinal axis,
at least one stabilizing web (6a, 6b) which has at least one cavity (14a, 14b) and which has an inner wall (9a, 9b) and outer wall (10a, 10b) and is wound in a spiral shape along the outer wall (4) of the hollow body (2) to form a winding distance (7a, 7b) and is connected to the hollow body (2), and
at least one heating wire (8a, 8b) which is covered towards the outside by a stabilizing web (6b), wherein
the stabilizing web (6b), in the region of the heating wire (8a, 8b), has a constant wall thickness on the side of the stabilizing web (6b) facing towards the lumen (5),
**characterized in that**
the hollow body (2) has a first layer (12a) facing towards the lumen (5) and a second layer (12b) facing away from the lumen.

2. Breathing tube according to Claim 1, **characterized in that** the stabilizing web (6b) has a constant wall thickness on the side of the stabilizing web (6b) facing away from the lumen (5).

3. Breathing tube according to the preceding claims, **characterized in that** the stabilizing web (6a, 6b), viewed in cross section, has a rectangular, square or part-circular shape.

4. Breathing tube according to the preceding claims, **characterized in that** the stabilizing web (6a, 6b) has at least one support web (13a, 13b).

5. Breathing tube according to Claim 4, **characterized in that** the support web (13a, 13b) passes through the cavity (14a, 14b), preferably centrally, and/or the support web (13a, 13b) has a constant dimension.

6. Breathing tube according to the preceding claims, **characterized in that** the wall thickness of the tube having the inner wall (3) and outer wall (4) is smaller than the wall thickness of the stabilizing web (6a, 6b) on the side facing towards the lumen (5).

7. Breathing tube according to the preceding claims, **characterized in that** the wall thickness of the tube having the inner wall (3) and outer wall (4) is in the range from 0.14 mm to 0.22 mm, preferably in the range from 0.16 mm to 0.20 mm.

8. Breathing tube according to the preceding claims, **characterized in that** the wall thickness of the stabilizing web (6a, 6b) on the side facing towards the heating wire (8a, 8b) is in the range from 0.7 mm to 1.2 mm, preferably in the range from 0.5 mm to 1.0 mm.

9. Breathing tube according to the preceding claims, **characterized in that** the hollow body (2) and the stabilizing web (6a, 6b) are made of the same material.

10. Breathing tube according to the preceding claims, **characterized in that** the material of the hollow body (2) is wound in each case in a spiral shape with an overlap region or in abutment.

11. Breathing tube according to the preceding claims, **characterized in that** the heating wire (8a, 8b) lies on the outer wall (4) of the hollow body (2) and is moreover enclosed by the outer wall (10a, 10b) of the stabilizing web (6a, 6b).

12. Breathing tube according to Claim 1 or 10, **characterized in that** the heating wire (8a, 8b) lies on the outer wall of the first layer (12a) of the hollow body (2) and is moreover enclosed by the second layer (12b) of the hollow body (2).

13. Breathing tube according to the preceding claims, **characterized in that** a first stabilizing web (6a) is wound in a spiral shape along the outer wall (4) of the hollow body (2) to form a winding distance (7a) along which at least two heating wires (8a, 8b) run, and/or
a second stabilizing web (6b) is wound in a spiral shape along the outer wall (4) of the hollow body (2) to form a winding distance (7b) along which at least two data lines (11a, 11b) run.

14. Breathing tube according to the preceding claims, **characterized in that** a distance between two adjacent stabilizing webs (6a, 6b) corresponds at least approximately to the width of a stabilizing web.

15. Method for producing a heatable breathing tube designed as a composite tube, with the following method steps:
winding an extruded flat band in a spiral shape around a mandrel (14), in order to form a hollow body (2) in the form of an elongate tube with inner wall (3) and outer wall (4), a longitudinal axis, and a lumen (5) formed by the inner wall (3) and extending along the longitudinal axis, wherein the hollow body (2) has a first layer (12a) facing towards the lumen (5) and a second layer (12b) facing away from the lumen,
winding an arrangement, consisting of at least two parallel heating wires (8a, 8b), in a spiral shape onto the outer surface of the hollow body (2),
winding an extruded stabilizing web (6b) in a spiral shape onto the outer surface of the hollow body (2) in the region of at least two parallel adjacent heating wires (8a, 8b), such that, after the at least two parallel heating wires (8a, 8b) have been wound onto the outer surface of the hollow body (2), the heating wires (8a, 8b) are surrounded on the outside by the stabilizing web (6b), and/or
winding an arrangement, consisting of at least two parallel two data lines (11a, 11b), onto the outer surface of the hollow body (2),
winding an extruded stabilizing web (6a) in a spiral shape onto the outer surface of the hollow body (2) in the region of at least two parallel adjacent data lines (11a, 11b), such that, after the at least two data lines (11a, 11b) have been wound onto the outer surface of the hollow body (2), the data lines (11a, 11b) are surrounded on the outside by the stabilizing web (6b), and
after the respective at least two heating wires (8a, 8b) and/or data lines (11a, 11b) have been wound onto the first layer (12a) of the hollow body (2), the remaining uncovered circumferential regions of the heating wires (8a, 8b) and/or data lines (11a, 11b) are embedded in the material of the second layer (12b) of the hollow body (2).

16. Method according to Claim 15, **characterized in that** the hollow body (2) is cooled before the at least two heating wires (8a, 8b) and/or at least two data lines (11a, 11b) are wound onto the outer surface of the hollow body (2).

17. Method according to Claim 15 or 16, **characterized in that**, after the respective at least two heating wires (8a, 8b) and/or data lines (11a, 11b) have been wound onto the outer surface of the hollow body (2), the remaining uncovered circumferential regions of the heating wires (8a, 8b) and/or data lines (11a, 11b) are embedded in the material of the stabilizing web (6a, 6b).

## Revendications

1. Tuyau respiratoire chauffable (1) qui est conçu comme un tuyau composite, ledit tuyau respiratoire comprenant
un corps creux (2) qui est enroulé en spirale pour former un tuyau allongé pourvu d'une paroi intérieure (3) et d'une paroi extérieure (4), d'un axe longitudinal et d'une lumière (5) formée par la paroi intérieure (3) et s'étendant le long de l'axe longitudinal,
au moins une nervure de stabilisation (6a, 6b) qui comporte au moins une cavité (14a, 14b), qui est pourvue d'une paroi intérieure (9a, 9b) et d'une paroi extérieure (10a, 10b) et qui est enroulée en spirale le long de la paroi extérieure (4) du corps creux (2) en formant un espace d'enroulement (7a, 7b) et reliée au corps creux (2), et
au moins un fil chauffant (8a, 8b) qui est recouvert extérieurement d'une nervure de stabilisation (6b),
la nervure de stabilisation (6b) présentant une épaisseur de paroi constante dans la zone du fil chauffant (8a, 8b) du côté de la nervure de stabilisation (6b) dirigé vers la lumière (5),
**caractérisé en ce que**
le corps creux (2) comporte une première couche (12a) dirigée vers la lumière (5) et une deuxième couche (12b) dirigé à l'opposé de la lumière.

2. Tuyau respiratoire selon la revendication 1, **caractérisé en ce que** la nervure de stabilisation (6b) présente une épaisseur de paroi constante du côté de la nervure de stabilisation (6b) qui est dirigé à l'opposé de la lumière (5).

3. Tuyau respiratoire selon les revendications précédentes, **caractérisé en ce que** la nervure de stabilisation (6a, 6b) a dans une vue en coupe transversale une forme rectangulaire, carrée ou partiellement circulaire.

4. Tuyau respiratoire selon les revendications précédentes, **caractérisé en ce que** la nervure de stabilisation (6a, 6b) comporte au moins une nervure de support (13a, 13b).

5. Tuyau respiratoire selon la revendication 4, **caractérisé en ce que** la nervure de support (13a, 13b) traverse la cavité (14a, 14b), de préférence au milieu, et/ou la nervure de support (13a, 13b) a une dimension constante.

6. Tuyau respiratoire selon les revendications précédentes, **caractérisé en ce que** l'épaisseur de paroi du tuyau pourvu de la paroi intérieure (3) et de la paroi extérieure (4) est inférieure à l'épaisseur de paroi de la nervure de stabilisation (6a, 6b) du côté qui est dirigé vers la lumière (5).

7. Tuyau respiratoire selon les revendications précédentes, **caractérisé en ce que** l'épaisseur de paroi du tuyau pourvu de la paroi intérieure (3) et de la paroi extérieure (4) est de l'ordre de 0,14 mm et 0,22 mm, de préférence de 0,16 mm à 0,20 mm.

8. Tuyau respiratoire selon les revendications précédentes, **caractérisé en ce que** l'épaisseur de paroi de la nervure de stabilisation (6a, 6b) du côté qui est dirigé vers le fil chauffant (8a, 8b) est de l'ordre de 0,7 mm à 1,2 mm, de préférence de l'ordre de 0,5 mm à 1, 0 mm.

9. Tuyau respiratoire selon les revendications précédentes, **caractérisé en ce que** le corps creux (2) et le nervure de stabilisation (6a, 6b) sont en le même matériau.

10. Tuyau respiratoire selon les revendications précédentes, **caractérisé en ce que** la matière du corps creux (2) est enroulée en spirale avec une zone de recouvrement ou en bout à bout.

11. Tuyau respiratoire selon les revendications précédentes, **caractérisé en ce que** le fil chauffant (8a, 8b) est en appui sur la paroi extérieure (4) du corps creux (2) et est par ailleurs enfermé par la paroi extérieure (10a, 10b) de la nervure de stabilisation (6a, 6b).

12. Tuyau respiratoire selon la revendication 1 ou 10, **caractérisé en ce que** le fil chauffant (8a, 8b) est en appui sur la paroi extérieure de la première couche (12a) du corps creux (2) et est par ailleurs enfermé par la deuxième couche (12b) du corps creux (2).

13. Tuyau respiratoire selon les revendications précédentes, **caractérisé en ce qu'**une première nervure de stabilisation (6a) est enroulée en spirale le long de la paroi extérieure (4) du corps creux (2) en formant un espace d'enroulement (7a) le long duquel s'étendent au moins deux fils chauffants (8a, 8b), et/ou
une deuxième nervure de stabilisation (6b) est enroulée en spirale le long de la paroi extérieure (4) du corps creux (2) en formant un espace d'enroulement (7b) le long duquel s'étendent au moins deux lignes de données (11a, 11b).

14. Tuyau respiratoire selon les revendications précédentes, **caractérisé en ce que** l'espace entre deux nervures de stabilisation (6a, 6b) adjacentes correspond au moins approximativement à la largeur d'une nervure de stabilisation.

15. Procédé de fabrication d'un tuyau respiratoire chauffable conçu comme un tuyau composite, ledit procédé comprenant les étapes suivantes :
enrouler en spirale une bande plate extrudée autour d'un mandrin (14) pour former un corps creux (2) sous la forme d'un tuyau allongé pourvu d'une paroi intérieure (3) et d'une paroi extérieure (4), d'un axe longitudinal et d'une lumière (5) formée par la paroi intérieure (3) et s'étendant le long de l'axe longitudinal, le corps creux (2) comportant une première couche (12a) dirigée vers la lumière (5) et une deuxième couche (12b) dirigée à l'opposé de la lumière,
enrouler en spirale un ensemble comprenant au moins deux fils chauffants parallèles (8a, 8b) sur la surface extérieure du corps creux (2),
enrouler en spirale une nervure de stabilisation extrudée (6b) sur la surface extérieure du corps creux (2) dans la zone d'au moins deux fils chauffants adjacents parallèles (8a, 8b) de sorte que, après avoir enroulé les au moins deux fils chauffants parallèles (8a, 8b) sur la surface extérieure du corps creux (2), les fils chauffants (8a, 8b) soient entourés extérieurement par la nervure de stabilisation (6b), et/ou
enrouler en spirale un ensemble comprenant au moins deux lignes de données parallèles (11a, 11b) sur la surface extérieure du corps creux (2),
enrouler en spirale une nervure de stabilisation extrudée (6a) sur la surface extérieure du corps creux (2) dans la zone d'au moins deux lignes de données adjacentes parallèles (11a, 11b) de sorte que, après l'enroulement des au moins deux lignes de données (11a, 11b) sur la surface extérieure du corps creux (2), les lignes de données (11a, 11b) soient entourées extérieurement par la nervure de stabilisation (6b), et
après avoir enroulé les au moins deux fils chauffants (8a, 8b) et/ou lignes de données (11a, 11b) sur la première couche (12a) du corps creux (2), noyer les zones périphériques restantes, non encore recouvertes, des fils chauffants (8a, 8b) et/ou des lignes de données (11a, 11b) dans la matière de la deuxième couche (12b) du corps creux (2).

16. Procédé selon la revendication 15, **caractérisé en ce que** le corps creux (2) est refroidi avant d'enrouler les au moins deux fils chauffants (8a, 8b) et/ou les au moins deux lignes de données (11a, 11b) sur la surface extérieure du corps creux (2).

17. Procédé selon la revendication 15 ou 16, **caractérisé en ce que**, après avoir enroulé les au moins deux fils chauffants (8a, 8b) et/ou lignes de données (11a, 11b) sur la surface extérieure du corps creux (2), les zones périphériques restantes, non encore recouvertes, des fils chauffants (8a, 8b) et/ou des lignes de données (11a, 11b) sont noyées dans la matière de la nervure de stabilisation (6a, 6b).
